# EUROPEAN PATENT APPLICATION

(11) **EP 1 652 886 A1**
(43) Date of publication of application: **03.05.2006**
(21) Application number: 04021917.2
(22) Date of filing: 15.09.2004
(51) Int. Cl.: C08L 71/02

(54) **Photocationic curable composition and new compound**

(71) Applicant: DAINIPPON INK AND CHEMICALS, INC., Itabashi-ku Tokyo (JP)
(72) Inventor: Lachowicz, Arthur, Dr., 13467 Berlin (DE); Gaudl, Kai-Uwe, Dr., 16540 Hohen Neuendorf (DE); Woelbing, Marion, 12683 Berlin (DE); Sato, Taiji, Yokohama (JP); Moriya, Kazuhiko, Saitama (JP); Yatsugi, Ken-ichi, Dr., Tokyo (JP)
(74) Representative: Albrecht, Thomas

(57) **Abstract**

The invention is related to a photocationic curable composition comprising
c) a compound having
   (a-1) an epoxy alicyclic structure selected from the group consisting of epoxy norbornene structure and 2,3-epoxy-1,4,5,8-dimethanonaphtalene structure, and
   (a-2) oxetane structure,
   wherein the epoxy alicyclic structure(a-1) is bonded to the oxetane structure(a-2) via a polyvalent organic group selected from the group consisting of an alkylene group, an alkylene ether group, and an alkylene thioether group, wherein the atom number between the structure(a-1) and the structure(a-2) is within the range of 1 to 8, or via a phenylene containing divalent organic group, and
d) a photoinitiator.

The cured product of the present invention exhibits high hardness. Therefore, the composition of the present invention is suitable for UV-inks, coatings, or adhesives.

## Description

### FIELD OF THE INVENTION

The present invention relates to a photocationic curable composition, which gives higher hardness after curing as UV-inks, coatings, or adhesives, and a new compound curable with a photocationic curing system.

### DESCRIPTION OF RELATED ART

Photocurable compositions, which can be hardened by a cationic mechanism, are widely used in the field of UV-inks, coatings, or adhesives in the last years due to their good adhesion to various substrates such as aluminium and plastics, low viscosity in the field of the coating materials, and their capability of curing without oxygen inhibition in contrast to free-radical curing.

Because of the practical merits, the photocurable compositions are particularly used in the graphic art field such as UV-flexo, UV-screen and UV-inkjet inks, industrial coating field such as coil coating, can coating and plastic coating, adhesives, release coatings such as UV silicone release coatings and dielectric coatings for encapsulation of transformer and LED.

Conventionally, the most common photocationic curable monomers as princial components in the photocurable composition were cycloaliphatic diepoxides, and commercially applied cycloaliphatic diepoxides in cationic curing compositions include, for example, 3,4-epoxy-cyclohexylmethyl-3,4-epoxycyclohexane carboxylate, bis-(3,4 epoxycyclohexyl)-adipate and limonene dioxide.

However, these conventional cycloaliphatic diepoxides inherently have low curing rates, therefore it was difficult to use the cycloaliphatic diepoxides in the graphic arts field or for coil coating or can coating applications, which require high curing rates.

As a means for overcoming the problem, there were proposed several monomers on behalf of the conventional cycloaliphatic diepoxides. For example, United State Patent No.6,166,101 discloses a composition of cycloaliphatic epoxide compound, mono-oxetane compounds, and a compound having a cyclohexylepoxide group and an oxetane group in the same molecule(column 9 line 23-40, formula 18), in order to improve curability, thereby, obtain a coating composition which easily cures even when low amount of UV irradiation.

The coating composition, however, still had a problem in that hardness of the obtained cured products resulted in quite inefficience. Therefore, the problem has been an obstacle for applying the composition into graphic inks, coil coatings or can coatings, because in these technical field, higher hardness such as abrasion resistance or scratch resistance are required.

### SUMMARY OF THE INVENTION

It is therefore an object of the invention to provide a photocationic curable composition having quite higher hardness in its cured product.

Another object is to provide a compound which can give the above mentioned higher hardness to the composition thereof.

Unexpectedly, we have now found that, with respect to chemical structure of photocationic curable monomers, by introducing epoxy alicyclic structure selected from epoxy norbornene structure or 2,3-epoxy-1,4,5,8-dimethanonaphtalene structure and oxetane structure simultaneously into its molecular structure, and boning them via a short distance aliphatic or aliphatic ether group or a phenyl containing group, a photocationic curable composition having excellent hardness in its hardened product can be obtained.

Accordingly, the present invention provides,
a photocationic curable composition comprising
a) a compound having
   (a-1) an epoxy alicyclic structure selected from the group consisting of epoxy norbornene structure and 2,3-epoxy-1,4,5,8-dimethanonaphtalene structure, and
   (a-2) oxetane structure,
   wherein the epoxy alicyclic structure(a-1) is bonded to the oxetane structure (a-2) via a polyvalent organic group selected from the group consisting of an alkylene group, an alkylene ether group, and an alkylene thioether group, wherein the atom number between the structure(a-1) and the structure (a-2) is within the range of 1 to 8, or via a phenylene containing divalent organic group, and
b) a photoinitiator.

The present invention also provides a new compound represented by the following chemical structure I, 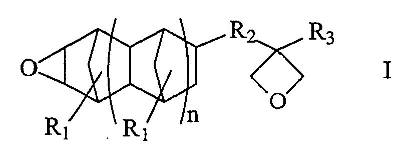 wherein R1 denotes methyl, ethyl, or hydrogen atom, R2 denotes a C1 to C8 alkylene, dimethylene ether, diethylene ether, R3 denotes a C1 to C4 alkyl, and n denotes 0 or 1.

According to the present invention, a photocationic curable composition can be provided, which gives high hardness to its cured product. Furthermore, a new compound can be provided, which is usable for the composition, and gives the aforementioned property to the composition.

Therefore, the composition of the present invention is suitable for UV-inks, coatings, or adhesives.

### DETAILED DESCRIPTION OF THE PREFERRED EMBODIMENTS

The embodiments of the present invention will hereinafter be described in detail.

The compound a), as one component of the composition of the present invention, is characterized in having
(a-1) an epoxy alicyclic structure selected from the group consisting of epoxy norbornene structure and 2,3-epoxy-1,4,5,8-dimethanonaphtalene structure, and
(a-2) oxetane structure.

Here, the epoxy norbornene structure can be represented, for example, by the following structure, 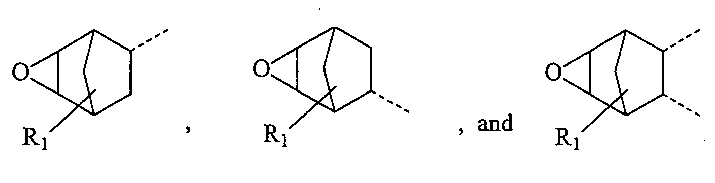 wherein the R₁ denote methyl, ethyl, or hydrogen atom, and dotted lines are bonds with the other structures.

On the other hand, the 2,3-epoxy-1,4,5,8-dimethanonaphtalene structure can be represented, for example, by the following structure, 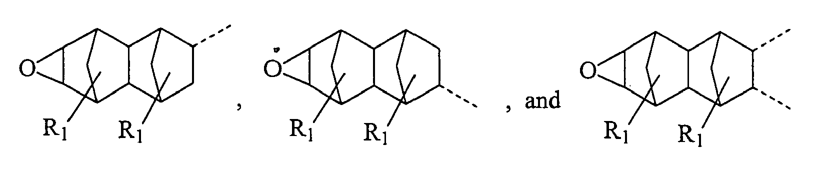 wherein R₁ denotes methyl, ethyl, or hydrogen atom, and dotted lines are bonds with the other structure.

The compound a) is furthermore characterized in that the epoxy alicyclic structure (a-1) is bonded to the oxetane structure (a-2) via a polyvalent organic group selected from the group consisting of an alkylene group, an alkylene ether group, and an alkylene thioether group, wherein the number of atom(s) between the structure(a-1) and the structure(a-2) is within the range of 1 to 8, or via a phenylene containing divalent organic group.

Examples of the alkylene group include methylene, ethylene, propylene, butylenes, 1,5-pentylene, and 1,6-hexene. Examples of the alkylene' ether group include dimethylene ether, diethylene ether, dipropylene ether, poly ether comprising these ether units. Finally, examples of the alkylene thioether group include dimethylene thioether, diethylene thioether, and dipropylene thioether.

In the present invention, as above mentioned, the number of atom(s) between the structure (a-1) and the structure (a-2) is within the range of 1 to 8, when the epoxy alicyclic structure(a-1) is bonded to the oxetane structure(a-2) via these alkylene groups, alkylene ether groups, and alkylene thioether groups. Namely, by selecting such a short length group, hardness of the cured product can be extremely enhanced. It is, therefore, that a range of 1 to 3 is particularly preferred.

In the case of selecting the phenylene containing divalent organic group as a linkage groups, the groups presented by the following formulas are preferable from the viewpoint of hardness of cured products thereof. Here, R₄ denotes methyl, ethyl, or hydrogen atom in the formulas.

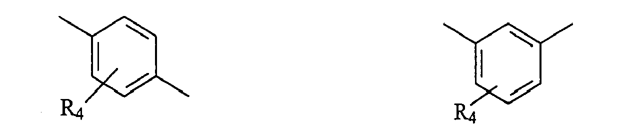

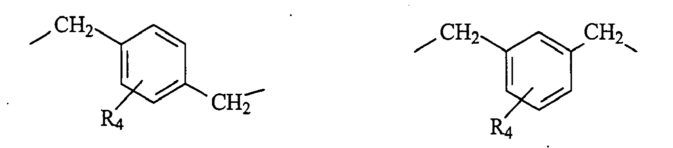

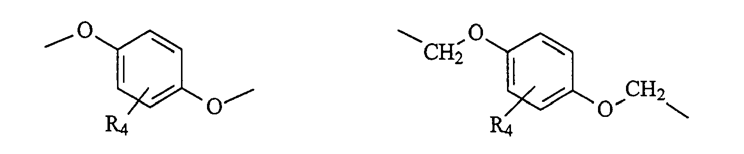

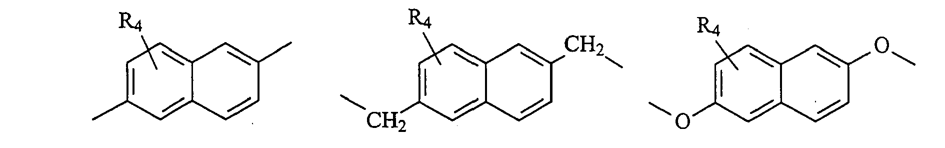

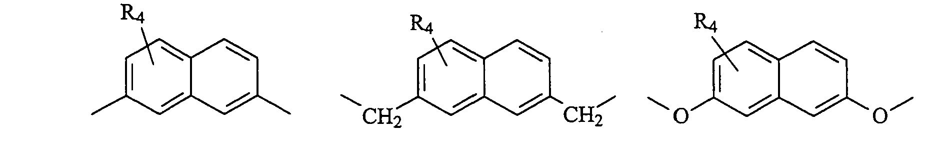

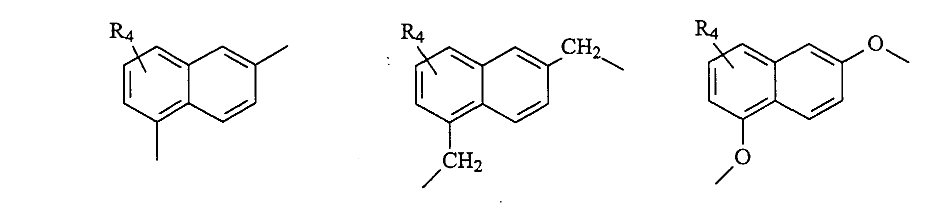

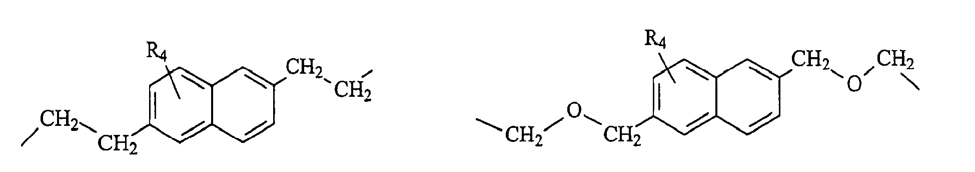

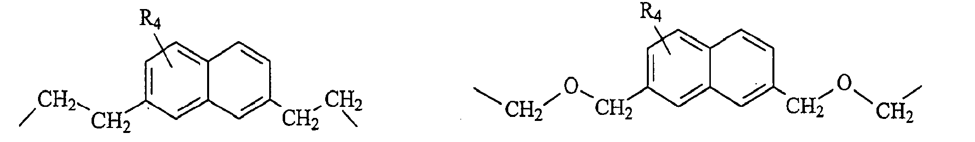

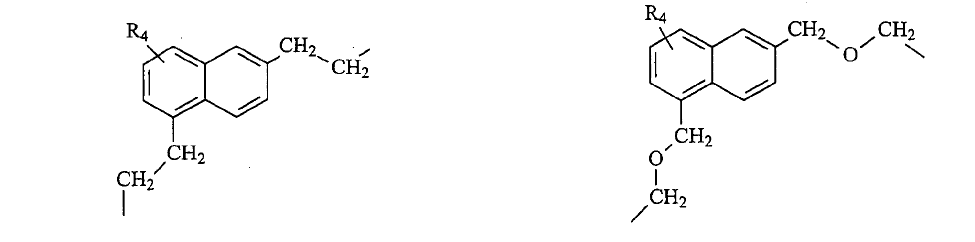

Accordingly, the examples of the compound a) having an epoxy alicyclic structure(a-1) and an oxetane structure(a-2), wherein the epoxy alicyclic structure (a-1) is bonded to the oxetane structure(a-2) via a polyvalent organic group selected from the group consisting of an alkylene group, an alkylene ether group, and an alkylene thioether group, wherein the number of atom(s) between the structure(a-1) and the structure(a-2) is within the range of 1 to 8, include the compounds having divalent groups represented by following formulas.

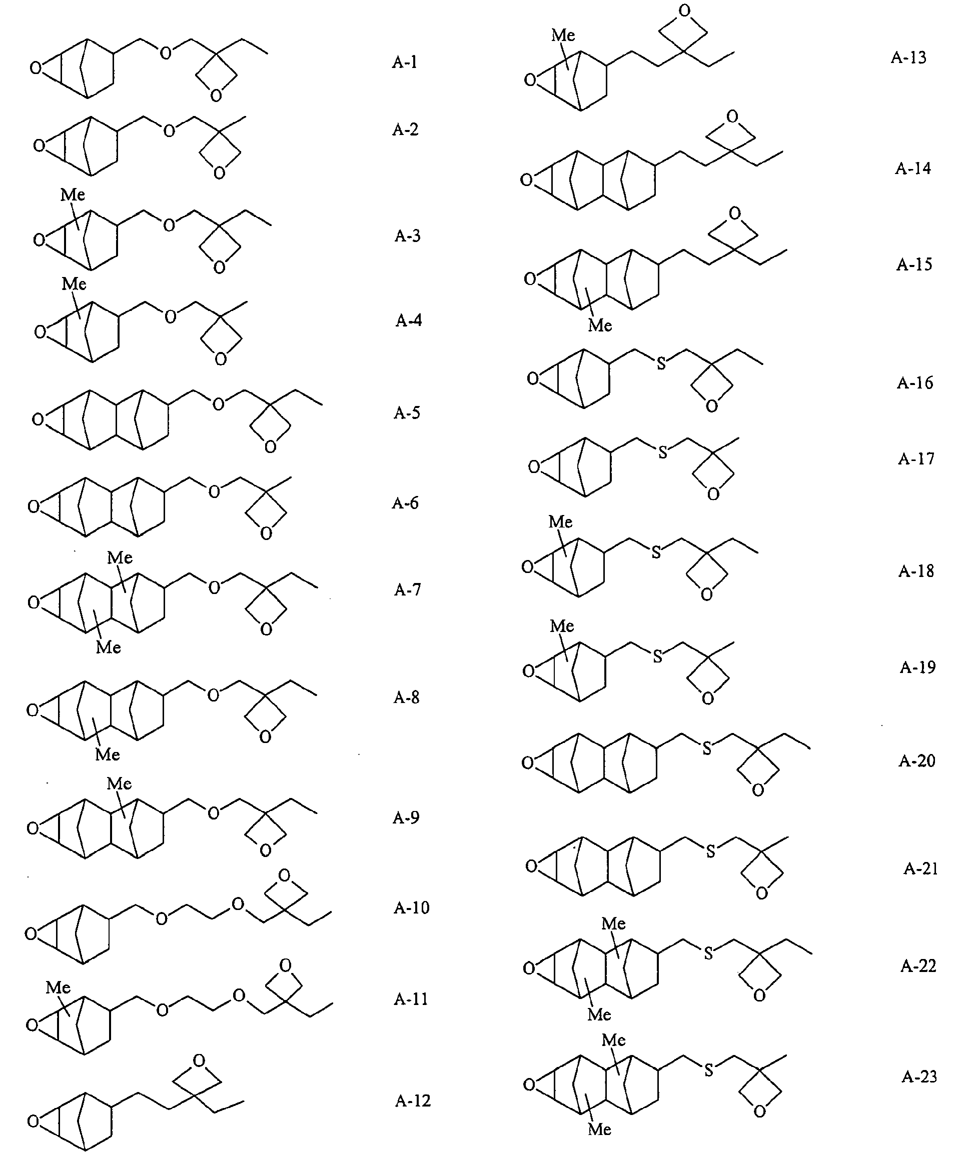

Examples of the compounds a) also include the compound having three functional groups per a molecule represented by the following formulas.

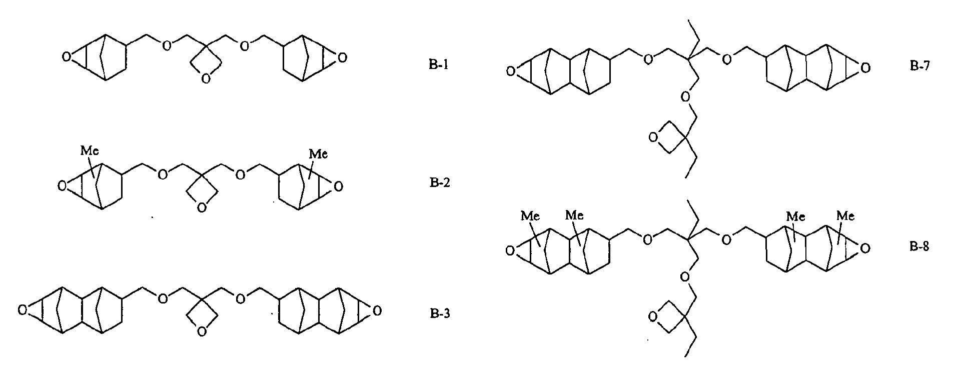

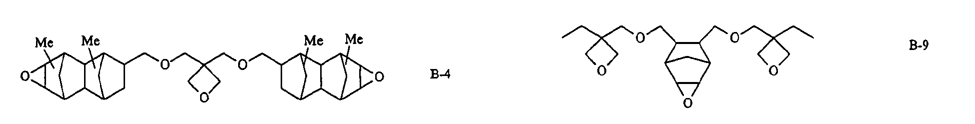

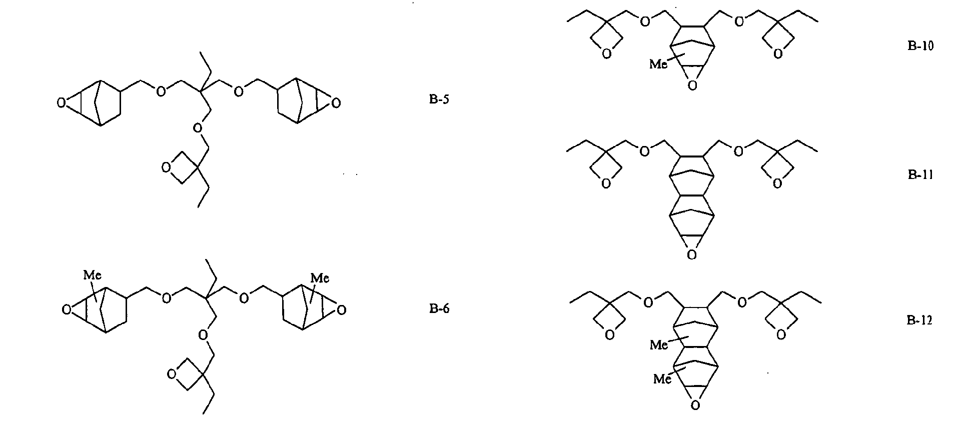

Furthermore, examples of the compound a) also include the compound having a phenylene containing divalent organic group represented by the following formulas.

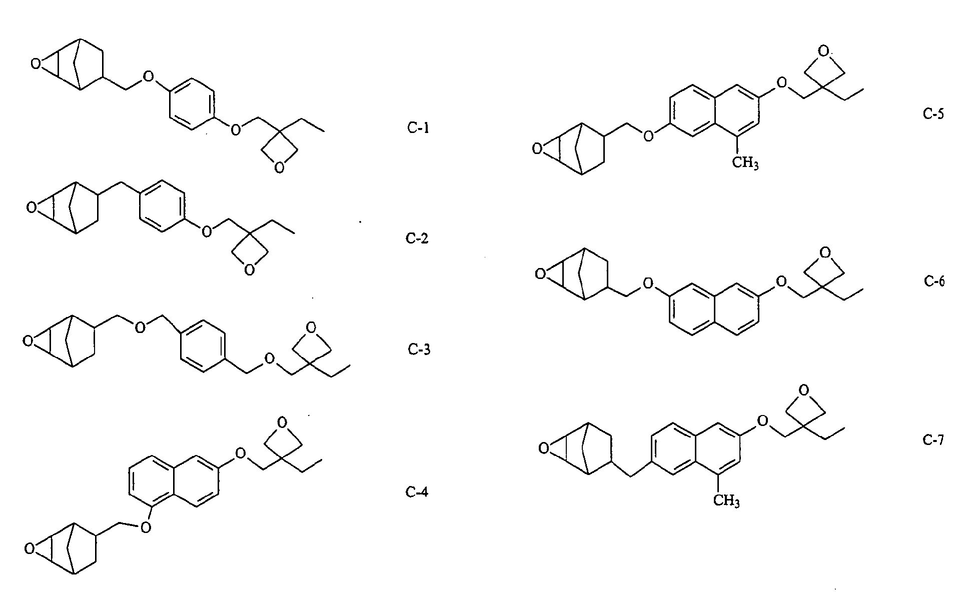

Among these compounds, the compounds a) having a linkage group having number of atom(s) within the range of 1 to 8, which are represented by the above A-1 to A22, and B-1 to B-12, are preferably used from the view point of superior hardness. Furthermore, these compounds have not only higher hardness, but also excellent adhesion to many types of substrates. Namely, in the case of that the structure (a-1) and the structure (a-2) are linking directly, which means the number of atom(s) is 0, only rigid cured products which show no adhesion to any substances and are no applicable for any use such as coatings or inks, are only given.

In general, hardness and adhesion are trade-off, because if a cured product is too rigid, decreasing of contacting area to a substrate is inevitable, and the cured product, thereby, results in exhibiting too low adhesion. Such a rigid cured product has no longer an inherent feature of photocationic cured products, because good adhesion is inherent technical merit of photocuring system. Therefore, having high hardness as well as good adhesion in the present invention is prominent technical effect in the field of photocationic curable composition.

In particular, as the aforementioned compound a), the compounds represented by the following formula I is preferable due to excellent hardness, 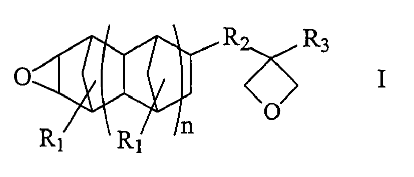 wherein R₁ denotes methyl, ethyl, or hydrogen atom, R₂ denotes a C1 to C8 alkylene, dimethylene ether, diethylene ether, R₃ denotes a C1 to C4 alkyl, and n denotes 0 or 1.

Examples of the compound represented by the formula I include the group which consists of the aforementioned A-1 to A-9, and A-12 to A-15. Furthermore, among these compound, from the viewpoint of hardness, a number of atom(s) of the R₂ in the formula I is even more preferably within a range of 1 to 3 as aforementioned.

Here, the compounds represented by formula I, may be also cured with a strong acid such as hexafluorophosphoric acid.

Furthermore, among these compounds shown in the formula I, the compounds represented by the following formula II are particularly preferable above all from the viewpoints of the hardness.

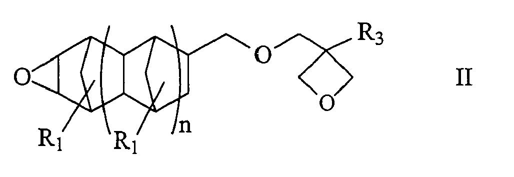

Here, R₁, R₃, and n are defined in formula I.

The compound represented by the formula II can be prepared by Diels-Alder reaction of allyloxetanylethers with (methyl)cyclopentadiene or its dimers followed by epoxidation.

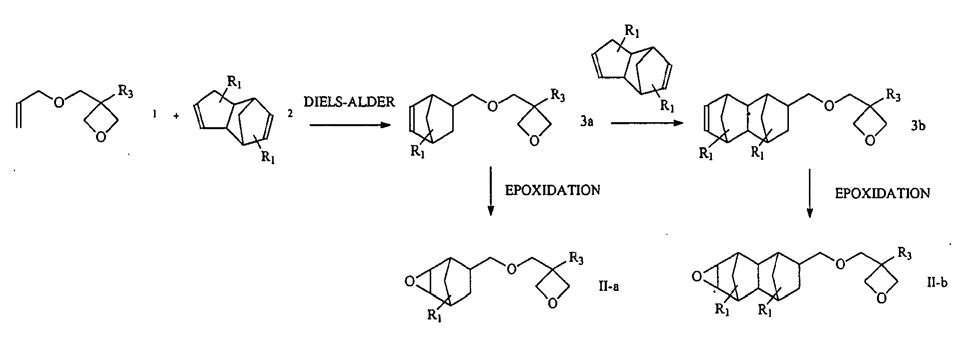

That is, the allyloxetanylethers of the general formula 1 are mixed with the cyclopentadiene itself or its derivatives 2, then heated to 60-220°C, preferably 150-170°C when cyclopentadiene dimer is used, and preferably 160-180°C when methylcyclopentadiene dimmer is used.

After a reaction time of 6 to 36 hours, preferably 8 to 24 hours, the reaction is stopped and the reaction mixture is purified by distillation, yielding the intermediates of the general formula 3a.

In order to give dimethanonaphtalene compounds such as A-5 to A-9, the compound represented by the formula 3a further is sujected to a reaction with the cylclopentadiene derivatives 2. The intermediates of the general formula 3a, or 3b, are epoxidized by standard epoxidation compounds such as hydrogen peroxide, Oxone (trademark of DuPont company), peracetic acid or perbenzoic acid. Preferred is peracetic acid due to cost and reactivity. The reaction temperature range is 0-80°C, preferred 10-30°C. After complete conversion, the products can be further purified by vacuum distillation over a column or short-way distillation.

As for the compounds other than the compound represented the aforementioned formula II, which compounds can be prepared by the conventional reaction process as shown in the following reaction scheme 1 to 7.

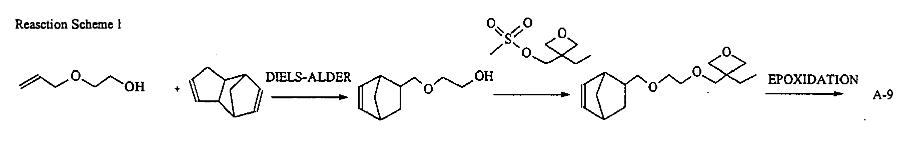

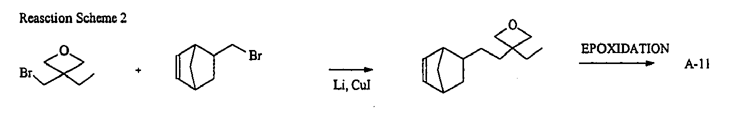

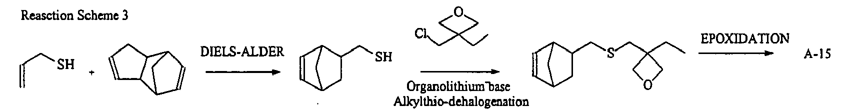

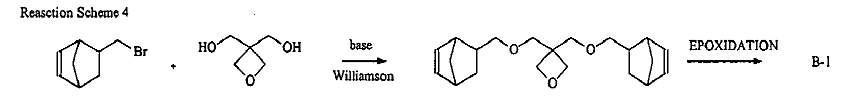

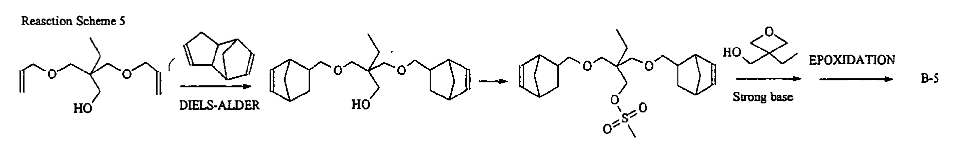

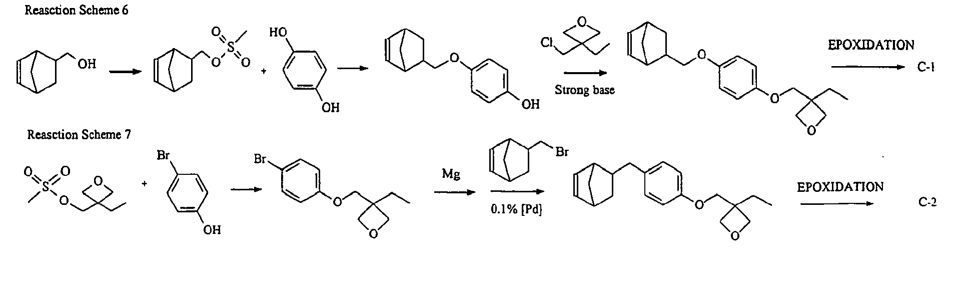

The photocationic curable composition of the present invention, as a curable monomer, may comprise the above-mentioned compound a) alone or may further comprise other photocationic curable monomers c) in combination.

Examples of the other photocationic curable monomers c) include conventional alicyclic epoxy compounds (c-1) such as dicyclopentadiene dioxide, limonene dioxide, di(3,4-epoxycyclohexyl methyl) adipate, (3,4-epoxy-cyclohexyl)methyl 3,4-epoxy-cyclohexanecarboxylate, (3,4-epoxy-6-methylcyclohexyl)methyl 3,4-epoxy-6-methylcyclohexanecarboxylate, ethylene-1,2-di(3,4-epoxycyclohexanecarboxylic acid)ester, the compounds represented by: 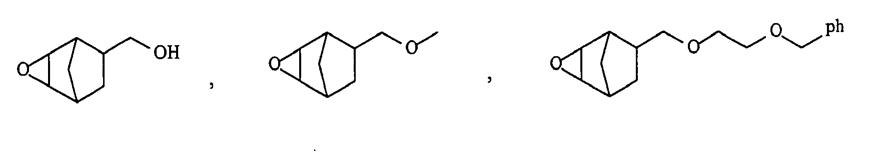 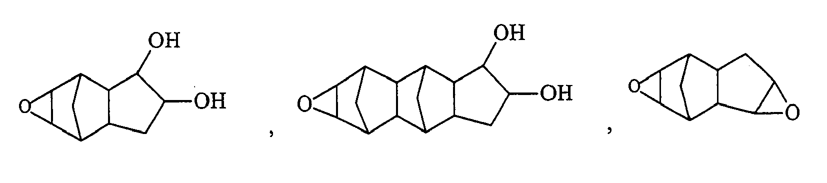 , and

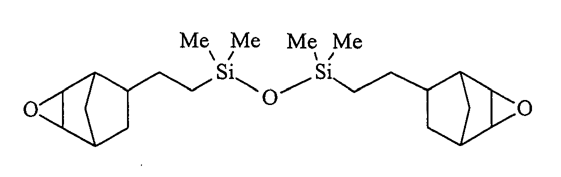

Preferred among these compounds (c-1), is (3,4-epoxycyclohexyl)methyl 3,4-epoxy-cyclohexanecarboxylate represented by following formula because of its curability, mechanical properties such as hardness and water resistance in the hardened products.

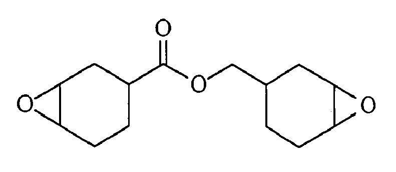

The other type of the photocationic curable monomers c) includes oxetane compound (c-2), for example, which can be represented by the following formulas:

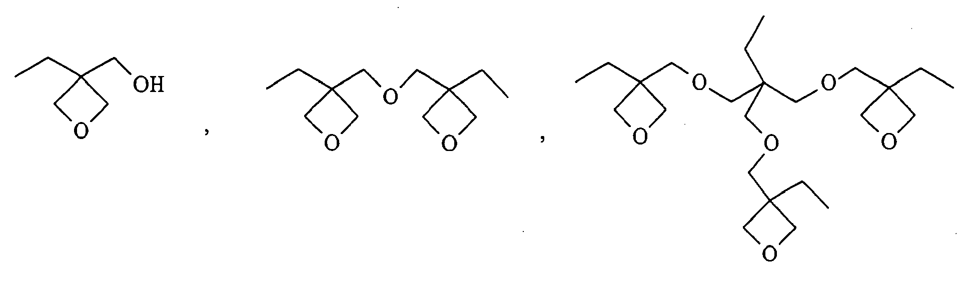

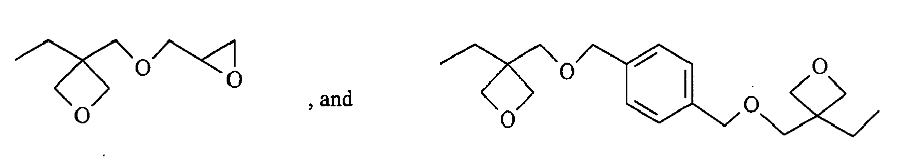

Preferred among these compounds (c-2) is 3-ethyl-3-(hydoroxymethyl)-oxetane represented by the following formula in light of photocurability.

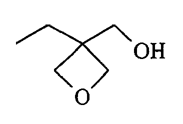

In the present invention, the other photocationic curable monomers c) can be used in amount of 80% or less based on the total amount of the present composition.

The photoinitiator b) which is used in the present invention may be a compound that generates cations when U.V. light is irradiated. Examples of the photoinitiator b) include aryl sulfonium salts and aryl iodonium salts. From the viewpoint of higher curability, and reactivity, preferred among them are aryl sulfonium salts, such as aryl sulfonium hexafluorophosphate and aryl sulfonium hexafluoroantimonate, and aromatic iodonium salts, such as diaryliodonium hexafluoroantimonate.

The amount of the photoinitiator b) used in the present invention is preferably within a range of 0.3 to 20 % by weight based on the total amount of the composition for a coating composition, and particularly in the field of clear coatings, a range of 2 to 6 % by weight is even more preferred. Moreover, a range of 4 to 12 % by weight is preferred for inks.

As above mentioned, the present composition can be advantageously applied to a field of UV-flexo, UV-screen, UV-inkjet inks, coil coating, can coating and plastic coating, adhesives, release coatings and dielectric coatings for chip encapsulation due to its superior hardness as well as adhesive.

In a case of preparing UV-flexo, UV-screen, or UV-inkjet inks, in addition to the above components, organic or inorganic pigments, and dispersants can be incorporated in the present composition. The dispersants assist in stabilising the dispersion of the pigment particles in the ink. Furthermore, it is possible to further use together, if necessary, additives such as plasticisers, dyes, biocides, antioxidants, viscosity modifiers, defoamers or any other additives as appropriate.

In a case of using as a coil coating, a can coating and a plastic coating, adhesives, release coatings or dielectric coatings, it is possible to further use together, if necessary, additives such as a lubricity-imparting agent; a sensitizer; an organic or inorganic pigment, a filler, a dye, a modifying resin such as polyol resin, phenolic resin, acrylic resin, polyester resin, polyolefin resin, epoxy resin, epoxidized polybutadiene resin; organic resin fine particles.

The composition of the present invention, as a coating, can be prepared by mixing the above-mentioned individual components and stirring them so as to be a uniform coating composition.

As a coating, the present composition can be coated on several substrates, such as a metal plate, a resin film-laminated metal plate, or a metal can. Here, the metal plate includes tinplate, aluminium, tin-free steel, iron, zinc, copper, zinc-plated steel, steel plated with an alloy of zinc and other metal. On the other hand, the resin film being laminated with metal plate includes polyester resin, such as polyethylene terephthalate, polyolefin resin, such as polyethylene or polypropylene, polyamide resin, epoxy resin, and polyvinyl chloride.

Curing process of the present composition can be carried out under ultraviolet or visible light within a range of 180 to 500 nm after printing or coating thereof. As light source for the curing process, ultraviolet light emitting bulbs are available in several designs, and in particular, microwave exited electrode-less bulbs are preferred.

The "Fusion H- bulb" is a typical UV source, consisting primarily of an electrical discharge in undoped mercury vapor. The doped "Fusion D-bulb" also contains a small amount of a metal halide. The "Fusion V-bulb" is similar to the "D" bulb but emits a larger fraction at longer wavelengths. Therefore, the appropriate bulb can be selected depending on the absorption spectrum of the photoinitiator.

This invention will be further explained by a consideration of the following non-limiting examples which are intended to be purely exemplary of this invention.

### Examples

### EXAMPLE 1

### [Synthesis of 6-(3-ethyl-oxetane-3ylmethoxymethyl)-3-oxa-tricyclo[3.2.1.0*4,2*]octane]

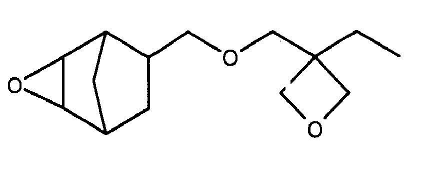

### Step 1: Synthesis of 3(bicyclo[2.2.1]hept-5-en-2ylmethoxymethyl)-3-ethyl-oxetane (Diels-Alder reation)

282g (1.8 moles) of 3-allyloxymethyl-3-ethyloxetane is mixed with 127g (0.96 mole) of cyclopentadiene dimer at room temperature. Then, the mixture is heated under nitrogen to 150-170°C. After 21 hours and a conversion of 90% the reaction is stopped and the product is purified by distillation over a 20cm vigreux column.

| | |
|---|---|
| Boiling point: | 90-95°C(0.01 hPa) |
| Yield: | 240g |
| Characterization: | 300 MHz-NMR [σ ,ppm, d-acetone]: |

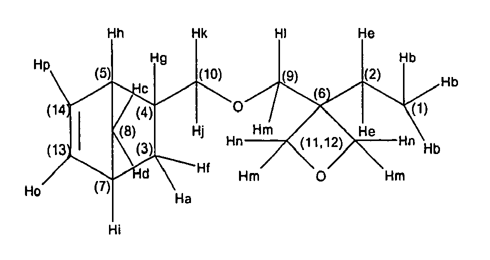

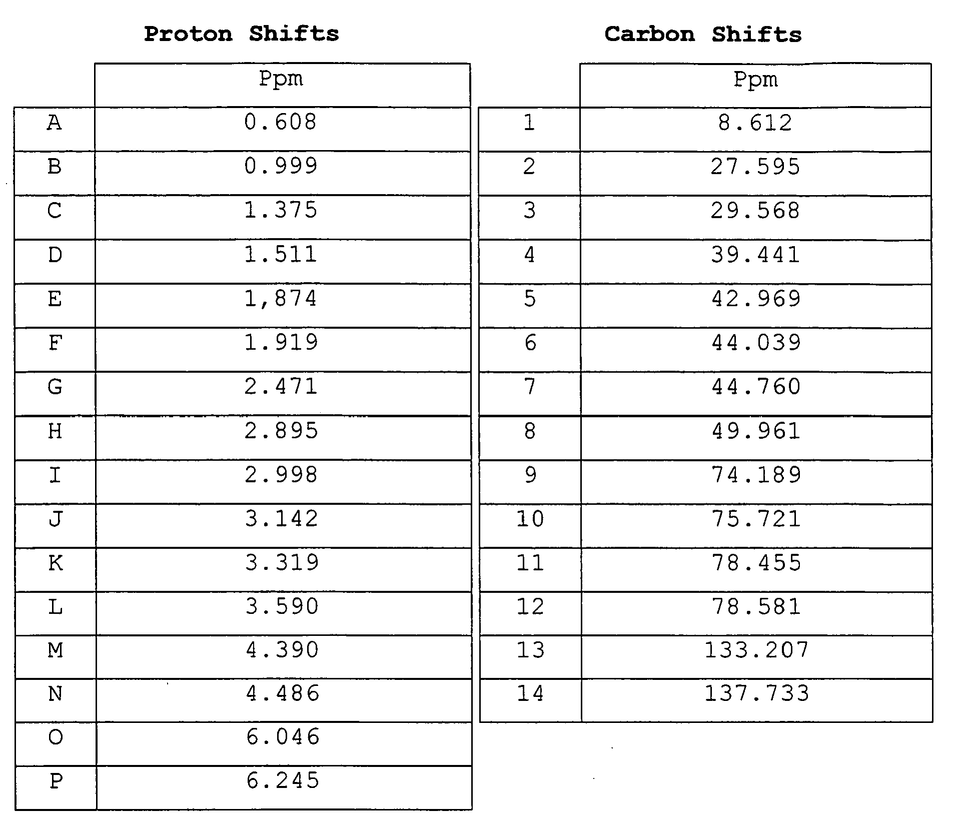

### Step 2: Epoxidation of 3-(bicyclo[2.2.1]hept-5-en-2-ylmethoxymethyl)-3-ethyl-oxetane

115.0g (0.46 mole) of m-chloroperbenzoic acid is dissolved in 800 ml of chloroform. The water-phase is separated. Then, a solution of 98g of 3-(bicyclo[2.2.1]hept-5-en-2-ylmethoxymethyl)-3-ethyl-oxetane in 100 ml of toluene is prepared. To that solution, which is cooled by an ice-bath, the chloroform solution is added in a way so that the reaction temperature does not exceed 15°C. The mixture is stirred for another hour at 10°C. Then, the precipitated m-chlorobenzoic acid is filtered off. The solution is shaken with a sodium bisulfite solution and sodium hydrogencarbonate solution, dried over sodium sulfate and distilled by a short-way distillation with a mantle temperature of 124°C at 0.01 hPa.

| | |
|---|---|
| Yield: | 63.5g |
| Purity: | 93% (measured by gas-chromatography) |
| Characterization: | Mass Spectroscopy [m/z]: 179(2), 139(25), 111(10), 93(41), 81(100), 79(31), 55(25), 41(60) 300 MHz-NMR [σ ,ppm, d-acetone]: |

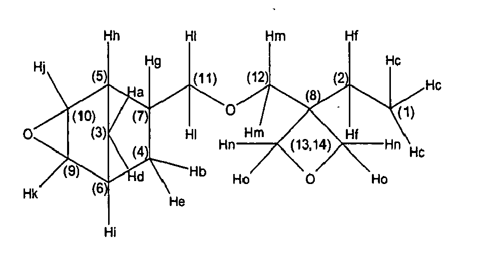

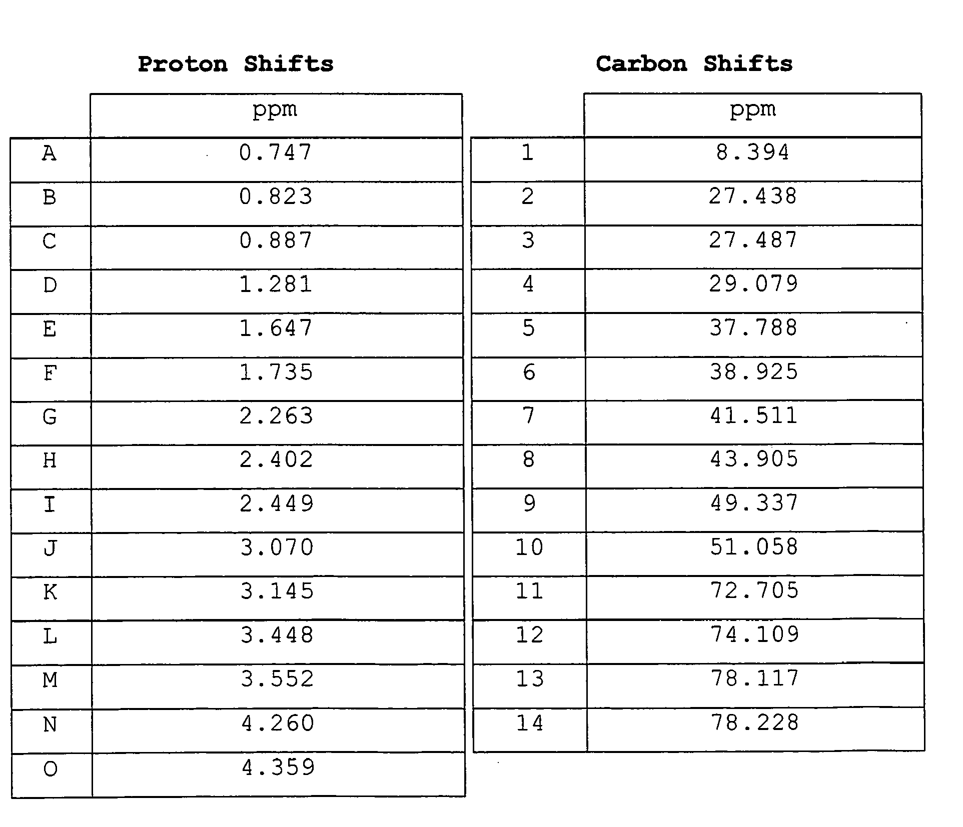

### Example 2 [Synthesis of 6-(3-ethyl-oxetane-3ylmethoxymethyl)-methyl-3-oxa-tricyclo[3.2.1.0*2,4*]octane]

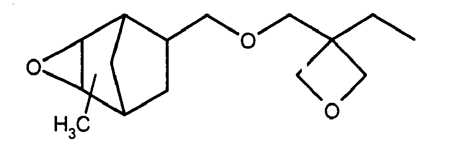

305.9g (1.96 moles) of 3-allyloxymethyl-3-ethyloxetane is mixed with 165.2g (1.03 moles) of methylcyclopentadiene dimer at room temperature. Then, the mixture is heated under nitrogen to 170°C-180°C. After 23 hours the reaction is stopped and the lower boiling materials are removed by evaporation at 40-50°C (5hPa). The product is purified by distillation.

| | |
|---|---|
| Boiling point: | 130°C (5 hPa) |
| Yield: | 323.0g |
| Characterization: | Mass Spectroscopy [m/z]: 238(M+), 223(2), 154(6), 97(11), 96(35), 95(84), 81(44), 69(35), 43(100), 41(64) |

### Step 2: Epoxidation of 3-(methyl-bicyclo[2.2.1]hept-5-en-2-ylmethoxymethyl)-3-ethyl oxetane.

The reaction is performed under the same conditions as in example 1, but using the product of the step 1.

| | |
|---|---|
| Yield : | 67g |
| Purity: | 94% (measured by gas-chromatography) |
| Characterization: | Mass Spectroscopy [m/z]: 252(M+), 153(30), 107(22), 96(35), 95(84), 81(44), 69(33), 57(25), 43(100), 41(64) |

### Comparative example [Synthesis of 3-(3-ethyl-oxetan-3ylmethoxymethyl)-7-oxabicyclo[4.1.0]heptane]

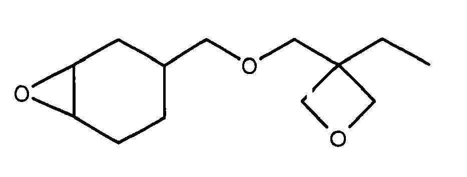

### Step 1: Synthesis of methanesulfonic acid cyclohex-3-enyl methylester

In a 2000cc. flask with a dropping funnel, a mechanical stirrer and a thermometer, is placed 125.0g (1.1 moles) of 3-cyclohexene methanol, 120.0g (1.2 moles) of triethylamine and 300cc. of diethylether. The flask is surrounded by an ice bad. Then, 116.0g (1.02 moles) of methane sulfonylchloride, dissolved in 200cc. diethylether is dropped slowly to the alcohol, so that the temperature does not exceed 15°C. When the addition is finished, the flask is connected with a reflux condenser and the mixture is stirred for another hour at 25°C. Then, the precipitated triethylammonium hydrochloride is filtered off. Then, 300cc. of petroleum ether is added to the solution for complete crystallisation. The second precipitation is filtered off as well and the solvents are removed under reduced pressure by a rotary evaporator. The residue is distilled under reduced pressure over a 20cm Vigreux column.

| | |
|---|---|
| Boiling point: | 55°C (0.03 hPa) |
| Yield: | 162.1g |
| Purity: | 98% (measured by gas-chromatography) |

### Step 2: Synthesis of 3-(cyclohex-3-enylmethoxymethyl)-3-ethyl-oxetane

125.0g (1.08 moles) of 3-ethyl-oxetanemethanol was dissolved in 500ml of toluene and sparged with nitrogen. Then, 24.0g (1 mole) of sodium hydride was added, so that the temperature did not exceed 60°C. After complete solution of the sodium hydride, the 155.0g of methanesulfonicacid cyclohex-3-enyl methylester was added and the mixture was stirred at 85°C under nitrogen, until the sulfonic methyl ester is consumed. Then, the reaction mixture is washed with 500ml of water, dried over anhydrous sodium sulfate and distilled und reduced pressure.

| | |
|---|---|
| Boiling point: | 77-82°C (0.02 hPa) |
| Yield: | 97g |
| Purity: | > 98% (GC) |
| Characterization: | ¹H-300-MHz-NMR [ σ , ppm, d-acetone]: 5.55 (s, 1H), 5.54 (s, 1H), 3.34(d, 2H), 4.24 (d, 2H), 3.41(s , 2H), 3.24 (d, 2H), 2.02 (m, 1H), 1.94 (2H, m), 1.79(m, 1H), 1.69 (m, 1H), 1.65 (m, 1H), 1.64 (m, 2H), 1.19(m, 1H), 0.78 (t, 3H) |

### Step 3: Epoxidaton of 3-(cyclohexyl-3-enylmethoxymethyl)-3-ethyloxetane

15.0g (0.046 mole) of m-chloroperbenzoic acid is dissolved in 80 ml of chloroform. The water-phase is separated. Then, a solution of 8.7g (0.041 mole) of 3-(bicyclo[2.2.1]hept-5-en-2-ylmethoxymethyl)-3-ethyl-oxetane in 10 ml of toluene is prepared. To that solution, which is cooled by an ice-bath, the chloroform solution is added in a way, so that the reaction temperature does not exceed 15°C. The mixture is stirred for another hour at 10°C. Then, the precipitated m-chlorobenzoic acid is filtered off. The solution is shaken with a sodium bisulfite solution and sodium hydrogencarbonate solution, dried over sodium sulfate. The solvents are removed under reduced pressure by a rotary-evaporator, and the product is distilled at 130°C at 0.01 hPa.

| | |
|---|---|
| Yield: | 6.6g |
| Purity: | 98% (measured by gas-chromatography) |
| Characterization: | Mass Spectroscopy [m/z]: 167(2), 129(3), 111(23), 110(24), 93(89), 81(79), 69(57), 55(79), 41(100). |

### [Pendulum Hardness Test]

The obtained compounds in accordance with Example 1, 2 and Comparative example, were mixed with cationic photoinitiator UVI 6990 (product of Dow Chemical company) as shown in the following table 1.

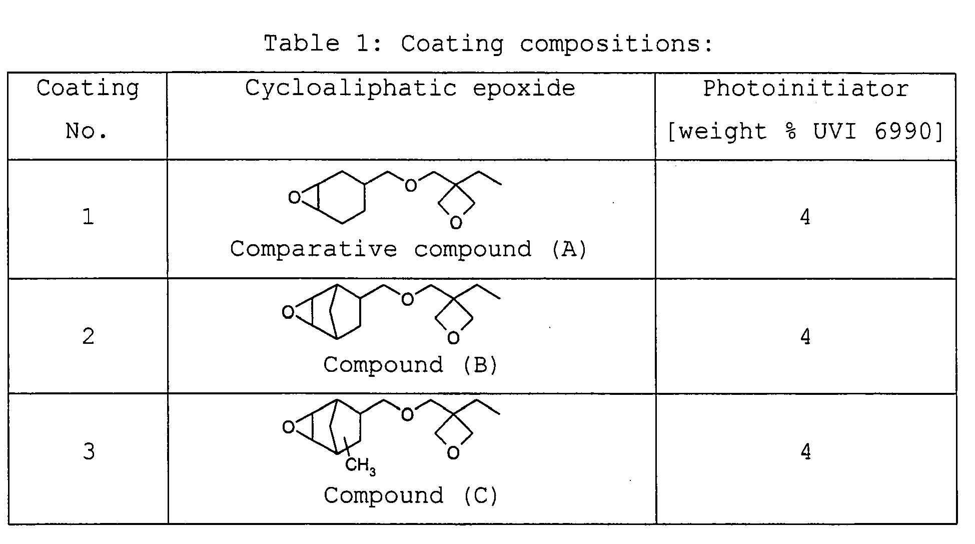

Then, these obtained coating compositions (Composition 1 to 3) were applied on top of a glass plate by a roller coater with a coating weight of 40g per square meter. The coatings on top of the glass plates were cured on a moving belt by UV-light with a Fusion H-bulb (120 W/cm) and a belt speed of 16m/min. Shortly after the curing, the coating samples became tack-free and were tested by pendulum hardness over a period of 24 hours.

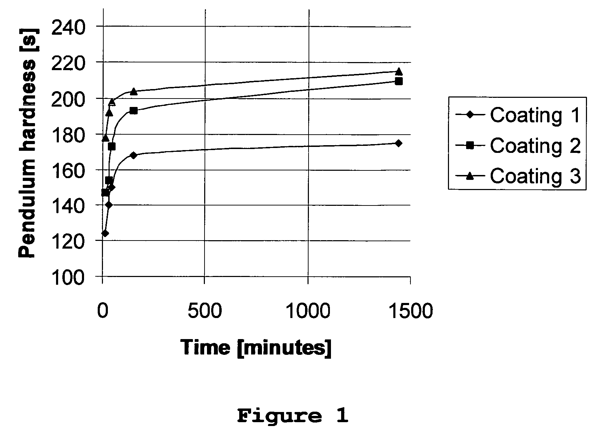

The results are shown in the following table 2, and are also illustrated in Fig.1 as well.

**Table 2**

| Time after UV-curing [minutes] | Pendulum Hardness Coating 1 [seconds] | Pendulum Hardness Coating 2 [seconds] | Pendulum Hardness Coating 3 [seconds] |
|---|---|---|---|
| 15 | 124 | 147 | 178 |
| 30 | 140 | 154 | 192 |
| 45 | 150 | 173 | 198 |
| 150 | 168 | 193 | 204 |
| 1440 | 175 | 210 | 215 |

As shown the results, the coating 2 and coating 3, containing norbornyl-epoxide structures of this invention, show higher hardness after tack-free cure and exhibit higher end-hardness after 24h, when dark-cure period is finished, compared to coating 1 containing the cyclohexylepoxide group.

## Claims

1. A photocationic curable composition comprising
a) a compound having
(a-1) an epoxy alicyclic structure selected from the group consisting of epoxy norbornene structure and 2,3-epoxy-1,4,5,8-dimethanonaphtalene structure, and
(a-2) oxetane structure,
wherein the epoxy alicyclic structure(a-1) is bonded to the oxetane structure(a-2) via a polyvalent organic group selected from the group consisting of an alkylene group, an alkylene ether group, and an alkylene thioether group, wherein the atom number between the structure(a-1) and the structure(a-2) is within the range of 1 to 8, or via a phenylene containing divalent organic group, and
b) a photoinitiator.

2. A photocationic curable composition according to claim 1, wherein the compound a) is represented by the following chemical structure I, 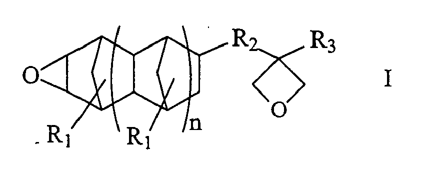 wherein R1 denotes methyl, ethyl, or hydrogen atom, R2 denotes a C1 to C8 alkylene, dimethylene ether, diethylene ether, R3 denotes a C1 to C4 alkyl, and n denotes 0 or 1.

3. A photocationic curable composition according to claim 1, wherein the composition further contains (3,4-epoxycyclohexyl)methyl 3,4-epoxy-cyclohexanecarboxylate or 3-ethyl-3-(hydroxymethyl)-oxetane.

4. A photocationic curable composition according to claim 1 or 2, the photoinitiator is an aryl sulfonium salts or an aromatic iodonium salts.

5. A photocationic curable composition according to claim 1, a concentration of the photoinitiator based on the total amount of the composition is within the range of 0.3 to 20 % by weight.

6. A new compound represented by the following chemical structure I, 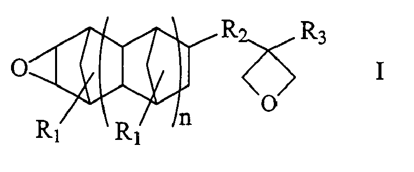 wherein R1 denotes methyl, ethyl, or hydrogen atom, R2 denotes a C1 to C8 alkylene, dimethylene ether, diethylene ether, R3 denotes a C1 to C4 alkyl, and n denotes 0 or 1.
